# EUROPEAN PATENT APPLICATION

(11) **EP 0 705 610 A1**
(43) Date of publication of application: **10.04.1996**
(21) Application number: 94115536.8
(22) Date of filing: 03.10.1994
(51) Int. Cl.: A61M 1/14, A61M 1/36

(54) **An integrated extracorporeal circulation system**

(71) Applicant: SORIN BIOMEDICA S.p.A., I-13040 Saluggia (Vercelli) (IT)
(72) Inventor: Vallana, Franco, I-10123 Torino (IT); Fini, Massimo, I-41037 Mirandola (Modena) (IT); Porro, Giampiero, I-22100 Como (IT)
(74) Representative: Bosotti, Luciano

(57) **Abstract**

An integrated extracorporeal circulation system including a plurality of devices chosen from a group including a cardiotome (2), a haemoconcentrator (3), a venous reserve (4), a heat exchanger (5), an oxygenator (6) and a pump (7, 8) integrated in an integrated assembly with flow lines (124, 134, 147, 175, 156) for the transfer of fluids between the various devices (2 to 8).

## Description

The present invention relates to extracorporeal circulation systems intended, for example, to take over the functions of the lungs and the circulation system during heart surgery requiring a heart-lung by-pass.
In the prior art, systems of the type specified above may essentially be described as individual-part systems, that is systems constituted by a plurality of devices (oxygenator, heat exchanger, pump, haemoconcentrator, etc. ) which were conceived as separate pieces of equipment intended to be connected to each other later, often in the operating theatre, so as to make up an extracorporeal circulation system.

Some manufacturers have attempted to design subassemblies formed, for example, by pieces of equipment mechanically connected to each other so as to form a single unit.

A typical example is a module which includes an oxygenator and a heat exchanger, such as, for example, the product sold under the trade name Monolyth by Bellco S.p.A of Mirandola, Italy.

However, these arrangements do not fully solve the inherent disadvantages of prior art systems.

These disadvantages include physiological problems, involving possible consequences for the patient undergoing extracorporeal circulation, especially if this is for a long period. These problems are largely due to the fact that in conventional extracorporeal circulation systems the various components, and above all the tubes used to connect them, have large areas in contact with the blood. The fact that there are large blood-system contact surfaces (surfaces which are foreign to the patient's body) may give rise to phenomena involving activation and/or alteration of the blood and of physiological liquids in general and having immunological and/or other consequences which could affect post-operative recovery. In general, the rather large overall volume of the tubes connecting the individual units means that on activating the system it is necessary to fill it with a corresponding volume of physiological solution, thereby negatively affecting the haemodilution.

In addition to the aforementioned physiological problems, there are logistical problems caused by the fact that the extracorporeal circulation system must, in effect, be assembled by the users, often in the operating theatre. This latter most often ends up cluttered (also) with the various elements of the extracorporeal circulation system and the connecting tubes thereof, often arranged in a lay-out which is anything but efficient. In addition, this last minute assembly, immediately prior to use, especially in the operating theatre, means that technicians end up resorting to operating arrangements, such as for example regulating the flow through some tubes by means of manually-controlled flow-restrictor devices or even surgical instruments which, while proving satisfactory certainly fail to satisfy the requirement for precise control and adjustment of a process (or rather a series of processes) such as a system for extracorporeal circulation. This applies especially when such regulation is intended to be controlled automatically or at least under the supervision of an automatic control unit.

The above applies equally to so-called "custom-pack" systems which the producers of extracorporeal circulation systems envisage producing, to satisfy specific requirements or to suit the surgical procedures of particular users or particular groups of users.

The object of the present invention is to provide a system which radically solves the aforesaid problems, especially in so far as the following aspects are concerned:
i) the possibility of producing a totally integrated extracorporeal circulation system which would minimise the effects of blood dilution while rationalising the operating lay-out and standardising the components of the systems;
ii) the possibility of producing an extracorporeal circulation system which proves totally non-traumatic, especially as far as the phenomena of haemolysis and platelet aggregation are concerned;
iii) the possibility of creating a system which is totally haemo-compatible with regard to the surfaces intended to come into contact with the blood, these surfaces being reduced to a minimum together with the consequent risk of introducing pollutants to the blood, and with regard to phenomena involving activation and/or alteration of the blood (such as, for example, activation of the coagulation process); and
iv) the possibility of producing a system which is automatically controlled and controllable so that it can adapt precisely and in real time to physiological functions in total safety, with the possibility of adopting an overall feedback control and adjustment arrangement dependent on sensors associated with the various components of the system.

According to the present invention this object is achieved by providing an extracorporeal circulation system having the characteristics specifically claimed hereinafter in the Claims.

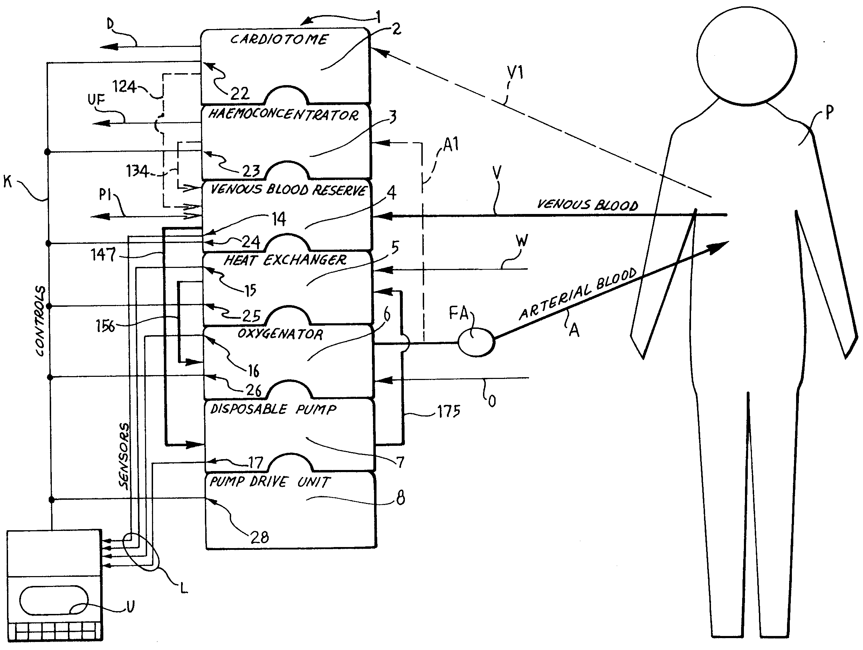

The invention will now be described, purely by way of non-limitative example, with reference to the appended drawing which schematically illustrates the structure of an extracorporeal circulation system according to the invention.

The system according to the invention, generally indicated 1, is characterised essentially in that it is an integrated system, constituted by a series of devices, indicated progressively from 2 to 8, coupled in a modular arrangement so as to form a single functional block with the tubes for conveying the treated fluids between the component devices 2 to 8 integrated inside it along with respective sensors 14, 15, 16, 17 and actuators (controls) 22, 23, 24, 25, 26, 28 connected to an overall system control unit indicated U and constituted, for example, by an electronic control unit such as a personal computer for example.

Overall then, the system 1 of the invention is constituted by an integrated system intended to be connected with the outside environment (patient P, fluids acting as servomeans such as water or air-and-oxygen, discharge lines, collection and infusion lines) through respective connections but with the transfer of the various treated fluids taking place inside the system 1 without the need for the users to arrange additional tubes, connections etc (especially in the operating theatre).

For the sake of clarity, it should be specified that the reference to ducts and flow lines extending "inside" the system should not be interpreted in the strictest sense whereby these ducts or lines are physically incorporated within the functional block constituting the system. Naturally, one or more of these ducts or lines may extend, at least in part, outside the system: what counts, for the purposes of the invention, is that the overall system be seen as a single, independent entity, generally supplied to the user ready for use and requiring no special operations beyond connection with the external environment (patient, servomeans, discharge, collection and infusion lines) as described above.

More accurately, the system 1 of the invention is intended to be connected to the patient's body by means of a venous blood collection line V and an arterial blood return line A. An arterial filter FA is normally interposed along the return line A while a further collection line V1 may be provided to act as cardiotome (drain), that is to vacuum blood from the surgical area.

As far as the connection to sources of the fluids acting as servomeans is concerned, this usually involves connection to a water supply line W, an air-and-oxygen supply line O and a vacuum line D (vacuum pump or the like).

The discharge lines generally include at least one ultrafiltrate discharge line UF (possibly with the capacity to regenerate this ultrafiltrate, using known methods).

Finally, connection is provided for collection and infusion lines PI.

One of the main advantages of the modular structure arises from the fact that the system of the invention lends itself to being easily assembled in various configurations, starting from a basic configuration which includes some essential elements (for example the oxygenator, the heat exchanger and the pump, with their respective control units) with the possible addition of auxiliary elements such as a venous reserve tank, haemoconcentrator, cardiotome etc.
In the configuration illustrated (purely by way of non-limitative example) in the drawing, the system of the invention includes the following devices, integrated in a single unit:
- a cardiotome 2 connected to the vacuum line D so as to be able to suck blood from the area of surgery through the line V1;
- a haemoconcentrator 3, which will be described in greater detail hereinafter;
- a reserve tank (venous blood reserve) 4 which collects the blood coming from the patient's circulation system through the line V as well as any blood removed by the cardiotome 2 or from the haemoconcentrator 3 through respective lines 124 and 134 integrated into the system, with the possibility of external exchange with withdrawal and infusion liquids via the lines PI.
- a heat exchanger 5 and a gas exchange module (oxygenator) 6 for the arterialization of blood taken from the venous reserve 4; the heat exchanger 5 is fluid-dynamically interposed between the venous reserve 4 and the oxygenator 6 so as to regulate the temperature of blood being oxygenated; and
- a pump chamber 7 (typically of the single-use or disposable type) with an associated actuator device (motor) 8 separably coupled to the pump 7 so as to allow the pump 7 to be replaced without necessarily having to replace the motor 8.

To be precise, the blood flows from the venous reserve 4 to the oxygenator 6 by means of a first line 147 though which blood from the venous reserve 4 (including that from the cardiotome 2 and the haemoconcentrator 3) passes through the pump 7 to be sent through a line 175 to the heat exchanger 5; from here the blood passes through a line 156 to the oxygenator 6, finally to be returned to the body of the patient P through the line A in which the arterial filter FA is located.

The lines 147, 175 and 156 are incorporated into the system like the lines 124 and 134. It may be seen from the diagram shown in the drawing that heat is exchanged in the exchanger 5 by means of water (thermostatically controlled - by known means, illustrated in the drawing - so as to keep the blood at the desired temperature) which is supplied through the line W, while the blood is oxygenated in the oxygenator 6 here again in known manner) using oxygen supplied through the line O.
The arterial blood which flows out from the system 1 through the line A may be partially drawn off through a line A1 to be sent to the input of the haemoconcentrator 3 where ultrafiltration is carried out. The ultrafiltrate is expelled from the system through the line UF while - as seen earlier - the blood purified by haemoconcentration flows through the line 134 to the venous reserve 4.

It must be emphasized once again that the various treatments described above are carried out according to principles well known to those skilled in the art, so their detailed description here would be entirely superfluous.

The various sensors 14 to 17 and actuators 22 to 28 integrated into the system enable the detection of parameters enabling the system to be controlled automatically through the control unit U. As a general rule, the following sensors are provided:
- a sensor, or preferably an arrangement of sensors 14 for measuring the blood level in the venous reserve and the haemochemical parameters of the venous blood,
- a temperature sensor 15 associated with the heat exchanger 5 (usually on the water side),
- a sensor 16 associated with the oxygenator 6 for measuring the oxygenation level and the removal of carbon dioxide, and
- a flow rate sensor 17 associated with the pump 7.

By means of lines generally indicated L, these sensors provide the control unit U with signals which when processed, according to known criteria which do not require a description here, enable the control unit U to emit, over a network of lines generally indicated K, corresponding signals controlling the actuators 22 to 28 which regulate (control) the quantities which determine the gaseous exchanges of heat and mass in the system.

It is possible, for example, to envisage the use of the following actuators:
- an actuator 22 for controlling the suction power of the cardiotome 2,
- an actuator 23 for regulating the rate at which ultrafiltration takes place in the haemoconcentrator 3,
- an actuator 24 for regulating the flow of venous blood from the patient to the venous reserve 4,
- an actuator 25 for controlling the flow rate of water (acting as diathermic liquid) in the heat exchanger 5',
- an actuator 26 for regulating the flow and composition of the gas flowing through the oxygenator 6, and
- an actuator 28 which controls the speed of the motor 8 associated with the pump 7.

It will be appreciated that, in the drawing, the connections between the various functional units have been indicated schematically by different lines. In particular, thick solid lines have been used for the main blood routes, broken lines for auxiliary blood routes, medium solid lines for auxiliary fluid connections and fine solid lines for the connections of sensors and actuators.

To summarise: the main characteristics of the system as described are the following.

Except for the control unit U, some sensors, the actuator lines and the pump control unit (motor), the system is suited to being constituted by single-use modules which can be replaced for each treatment.

The system of the invention enables the number of connections between the various components forming the system to be drastically reduced, thanks to the integration of these components, thus enabling the overall volume to be reduced, meaning that the patient's blood is less diluted.

It is possible to configure the system according to the user's specifications during its manufacture, thus making it possible to verify and guarantee the overall efficiency. This does not occur today for the majority of extracorporeal circulation systems currently in use, the components of which are separate, may have come from different suppliers and may be functionally and operationally incompatible.

It is possible to monitor/control the fundamental parameters of the extracorporeal circulation with the use of predetermined optimised algorithms, implemented by the control unit U.

The extreme compactness of the system fully rationalises the lay-out in the operating theatre.

Finally, it is possible to tailor final assembly operations according to the specific requirements of the user thanks to the use of components/devices which are specially designed to be integrated with each other.

In particular, as shown schematically in the drawing, the various components 2 to 8 are advantageously made such that their respective casings are mechanically connectable, as shown in the drawing where each of the elements of the system (except for the cardiotome 2) has a tenon element provided to mechanically engage a corresponding cavity in an element coupled to it. Naturally, this illustration is only schematic, taking into account the fact that in reality the shape and dimensions of the various elements 2 to 8 are usually rather different from each other. The general characteristic of mechanical connectability remains valid, as does the ordered connection sequence in which the various parts, indicated in the drawing by progressive reference numbers 2 to 8, are connected to each other. While not essential, this ordered sequence must be considered preferential from the point of view of the overall functioning of the system, especially when considering the possibility of configuring the system as a basic assembly including the essential modules (venous reserve, oxygenator, heat exchanger and pump) with the other devices (cardiotome, haemoconcentrator) provided as auxiliary modules, arranged selectively according to the requirements of the user. In particular, it must be made clear that in addition to the configuration illustrated, the system of the invention may have further elements integrated into it (for example elements for the infusion or controlled release of drugs and the like).

Naturally, the principle of the invention remaining unchanged, manufacturing details and embodiments may vary widely from those described and illustrated without departing thereby from the scope of the present invention.

## Claims

1. An extracorporeal circulation system characterised in that it comprises an integrated assembly having a plurality of devices chosen from a group comprising:
- a cardiotome (2),
- a haemoconcentrator (3),
- a venous reserve (4),
- a heat exchanger (5),
- an oxygenator (6), and
- a pump (7, 8).

2. A system according to Claim 1, characterised in that the flow lines (124, 134, 147, 175, 156) for the transfer of fluids between the said devices (2 to 8) are also integrated into the said assembly.

3. A system according to Claim 1 or Claim 2, characterised in that the devices forming the said assembly have casings shaped so that they can be mechanically connected so as to form the said integrated assembly.

4. A system according to any of Claims from 1 to 3, characterised in that the said devices have associated sensors (15, 16, 17) and/or actuators (22, 23, 24, 25, 26, 28) and in that a control unit (U) is associated with the system and connected to the said sensors and/or the said actuators in order to control the operation of the system (1) automatically.

5. A system according to any preceding Claim, characterised in that one of the devices of the said assembly is a pump (7,8) including a single-use pumping module (7) connected with a motor (8) which can be associated with different systems in succession.

6. A system according to Claim 1, characterised in that the said assembly includes a plurality of the said devices arranged in the sequence given in Claim 1.
